# EUROPEAN PATENT APPLICATION

(11) **EP 2 412 297 A1**
(43) Date of publication of application: **01.02.2012**
(21) Application number: 10755972.6
(22) Date of filing: 18.03.2010
(51) Int. Cl.: A61B 1/00

(54) **COVER TYPE ENDOSCOPE FOR TREATMENT AND COVER FOR ENDOSCOPE**

(30) Priority: 25.03.2009 JP 2009074087
(71) Applicant: Olympus Corporation, Shibuya-ku Tokyo 151-0072 (JP)
(72) Inventor: ITO, Yoshiaki, Hachioji-shi Tokyo 192-8512 (JP); TANAKA, Hirokazu, Hachioji-shi Tokyo 192-8512 (JP); KITAGAWA, Hideya, Hachioji-shi Tokyo 192-8512 (JP)
(74) Representative: Winter, Brandl, Fürniss, Hübner, Röss, Kaiser, Polte - Partnerschaft
(86) International application number: PCT/JP2010/054690
(87) International publication number: WO 2010/110174

(57) **Abstract**

A cover-adapted treatment endoscope (10) includes an endoscope cover (13) which covers an endoscope main body (21). The endoscope cover (13) includes a cover insertion portion main body (25), the cover insertion portion main body (25) including an insertion passage (20) into which the endoscope main body (21) is inserted, a cover distal end portion (91) provided at a most distal side portion, and a cover bending portion (92) which is provided to the proximal side of the cover distal end portion (91) and which is configured to be bent in response to the bending of a main body bending portion (33) of the endoscope main body (21) in an attached condition where the endoscope main body (21) is inserted in the insertion passage (20), a positioning mechanism (139) configured to determine an insertion position of the endoscope main body (21) where the main body distal portion (32) does not project from a distal end of the cover insertion portion main body (25) in the attached condition, a coupling portion (27) which couples the endoscope main body (21) to the cover insertion portion main body (25) in the attached condition, and one or more treatment arm/arms (45, 46) which projects/project from the distal end of the cover insertion portion main body (25) toward a distal side and each of which includes a bending mechanism (101, 102).

## Description

### Technical Field

The present invention relates to a cover-adapted treatment endoscope in which an endoscope insertion portion is covered with an endoscope cover when used, and the endoscope cover used in the cover-adapted treatment endoscope.

### Background Art

Recently, it has been often the case that an endoscope is used to perform an operation in a body cavity such as stomach or large intestine so that the burden on a patient can be reduced. In this case, a treatment endoscope having a treatment function that enables a low invasive operation is used.

Patent Literature 1 has disclosed a treatment endoscope which includes a bending portion and a flexible tube and which is provided with one or more arm/arms each of which has a bending function to project from the distal end of an insertion portion main body. This treatment endoscope is used not only to observe a body cavity but also to conduct a treatment such as cutting of an affected part within the body cavity with the arm/arms having the bending function.

Patent Literature 2 has disclosed an over tube which guides, into a body cavity, endoscope insertion portions that are housed together. That is, a plurality of instruments housed in the over tube are guided into the body cavity.

Patent Literature 3 has disclosed a cover-adapted endoscope. In this endoscope, a cover is attached to and covers an endoscope insertion portion, and the cover removed after use is disposed of. This cover-adapted endoscope reduces the trouble of cleaning and sterilizing the endoscope.

### Citation List

### Patent Literature

Patent Literature 1: Jpn. Pat. Appln. KOKAI Publication No. 2005-095590
Patent Literature 2: Jpn. Pat. Appln. KOKAI Publication No. 2005-325303
Patent Literature 3: Jpn. Pat. Appln. KOKAI Publication No. 7-299024

### Summary of Invention

### Technical Problem

The treatment endoscope according to Patent Literature 1 includes the arms configured to conduct a treatment such as cutting of an affected part within the body cavity, so that a distal end portion of an endoscope insertion portion in which the arms are disposed has a complex configuration. The distal end portion of the endoscope insertion portion having the complex configuration is basically a part that tends to be contaminated with blood or the like. Therefore, as compared with general endoscopes, the cleaning and sterilization of the distal end portion of the endoscope insertion portion is much more troublesome. Accordingly, the cleaning and sterilization require much labor and time, and the burden on a user and maintenance costs increase.

The device configuration according to Patent Literature 2 is complex because the plurality of instruments housed in the over tube are guided. Moreover, instruments including the endoscope insertion portions are individually cleaned and sterilized, so that the cleaning and sterilization are much more troublesome than in general endoscopes. Accordingly, the cleaning and sterilization require labor and time, and the burden and maintenance costs increase.

The cover-adapted endoscope according to Patent Literature 3 reduces the trouble of cleaning and sterilizing the endoscope. However, in this cover-adapted endoscope, when an arm having the bending function is provided in the insertion portion to be inserted in an endoscope cover, the insertion portion that includes the arm is covered with the endoscope cover. Therefore, it is extremely difficult to ensure the efficiency in the insertion of the insertion portion into the endoscope cover. It is also difficult to adapt the endoscope cover without impairing the function of the endoscope insertion portion. Moreover, covering the insertion portion with the cover is also difficult.

The present invention has been made in view of the foregoing problems, and is directed to ensure the efficiency in the insertion of the insertion portion main body into the endoscope cover, and allow the insertion portion main body to be easily inserted into the endoscope cover and thus used, thereby achieving the effective use of the cover-adapted treatment endoscope.

### Solution to Problem

According to one aspect of the invention, a cover-adapted treatment endoscope includes that an endoscope main body which includes a main body distal portion provided at a most distal side portion, and a main body bending portion provided to a proximal side of the main body distal portion and configured to bend; a main body operation portion which is provided to the proximal side of the endoscope main body and which is configured to operate the main body bending portion; and an endoscope cover which covers the endoscope main body, wherein the endoscope cover includes a cover insertion portion main body, the cover insertion portion main body including an insertion passage into which the endoscope main body is inserted, a cover distal end portion provided at a most distal side portion, and a cover bending portion which is provided to the proximal side of the cover distal end portion and which is configured to be bent in response to the bending of the main body bending portion of the endoscope main body in an attached condition where the endoscope main body is inserted in the insertion passage, a positioning mechanism configured to determine an insertion position of the endoscope main body where the main body distal portion does not project from a distal end of the cover insertion portion main body in the attached condition, a coupling portion which couples the endoscope main body to the cover insertion portion main body in the attached condition, one or more treatment arm/arms which projects/project from the distal end of the cover insertion portion main body toward a distal side and each of which includes a bending mechanism, and an arm operation portion configured to operate the treatment arm/arms.

According to one another aspect of the invention, an endoscope cover covers an endoscope main body of a cover-adapted treatment endoscope. The endoscope cover includes that a cover insertion portion main body, the cover insertion portion main body including an insertion passage into which the endoscope main body is inserted, a cover distal end portion provided at a most distal side portion, and a cover bending portion which is provided to the proximal side of the cover distal end portion and which is configured to be bent in response to the bending of the main body bending portion of the endoscope main body in an attached condition where the endoscope main body is inserted in the insertion passage; a positioning mechanism configured to determine an insertion position of the endoscope main body where the main body distal portion does not project from a distal end of the cover insertion portion main body in the attached condition; a coupling portion which couples the endoscope main body to the cover insertion portion main body in the attached condition; one or more treatment arm/arms which projects/project from the distal end of the cover insertion portion main body toward a distal side and each of which includes a bending mechanism; and an arm operation portion configured to operate the treatment arm/arms.

### Advantageous Effects of Invention

According to the present invention, the treatment arm/arms is/are provided to the side of the endoscope cover, so that the efficiency in the insertion of the endoscope main body into the endoscope cover is easily ensured in the cover-adapted endoscope. Moreover, the treatment arm/arms is/are provided to the side of the endoscope cover that can be removed from the endoscope main body, so that the cleaning of the cover-adapted endoscope can be simpler. Further, endoscope covers having various shapes and configurations and including a distal side bending portion and the arm/arms can be used in a common endoscope main body, and various forms of treatment endoscopes can be easily obtained.

### Brief Description of Drawings

FIG. 1 is a perspective view showing a treatment endoscope according to a first embodiment of the present invention in which a cover-adapted endoscope and an endoscope cover are separated from each other;
FIG. 2 is a perspective view showing the treatment endoscope according to the first embodiment in which the cover-adapted endoscope and the endoscope cover are coupled to each other;
FIG. 3 is a perspective view showing the configuration of a proximal end portion of the endoscope cover of the treatment endoscope according to the first embodiment;
FIG. 4 is a perspective view showing the cover-adapted endoscope of the treatment endoscope according to the first embodiment immediately before inserted into the endoscope cover;
FIG. 5 is a perspective view showing the cover-adapted endoscope of the treatment endoscope according to the first embodiment;
FIG. 6A is a perspective view showing the configuration of a main body bending portion of an endoscope main body of the cover-adapted endoscope according to the first embodiment;
FIG. 6B is a front view showing one bending piece that constitutes the main body bending portion of the endoscope main body according to the first embodiment;
FIG. 7 is a perspective view showing a distal end portion of a cavity insertion portion of the endoscope cover according to the first embodiment in which arms are in a waiting posture;
FIG. 8 is a perspective view showing the distal end portion of the cavity insertion portion of the endoscope cover according to the first embodiment in action in which the arms are outstretched;
FIG. 9 is a perspective view showing a distal end portion of the cavity insertion portion of the endoscope cover according to a first modification of the first embodiment in action in which the arms are outstretched;
FIG. 10 is a front view of a distal end portion of the endoscope cover of the treatment endoscope according to the first embodiment;
FIG. 11 is a longitudinal sectional view of the distal end portion of the endoscope cover along the line A-A of FIG. 10;
FIG. 12 is a longitudinal sectional view of the distal end portion of the endoscope cover along the line B-B of FIG. 10;
FIG. 13 is a sectional view showing, in a magnified form, a part indicated by C in FIG. 12;
FIG. 14 is a perspective view showing a cover main body of the endoscope cover according to the first embodiment viewed from a proximal side;
FIG. 15A is a partially sectional side view showing a connecting/disconnecting structure which connects/disconnects a wire of the treatment endoscope according to the first embodiment partway;
FIG. 15B is a plan view only showing one end of the connecting/disconnecting structure which connects/disconnects the wire of the treatment endoscope according to the first embodiment partway;
FIG. 16 is a side view showing the connecting/disconnecting structure which connects/disconnects the wire of the treatment endoscope according to the first embodiment partway in a state that the wire is coupled;
FIG. 17 is a schematic diagram showing a coupling structure of a connecting/disconnecting portion of wires of the treatment endoscope according to a second modification of the first embodiment;
FIG. 18A is a side view showing the connecting/disconnecting structure of the wire of the treatment endoscope according to a third modification of the first embodiment in a state that before a connection chip is inserted into a connector;
FIG. 18B is a side view showing the connecting/disconnecting structure of the wire of the treatment endoscope according to the third modification of the first embodiment in a state that the connection chip is inserted in the connector at right angles;
FIG. 18C is a side view showing the connecting/disconnecting structure of the wire of the treatment endoscope according to the third modification of the first embodiment in a state that the connection chip is tilted so that the axial center of the connection chip is aligned with the axial center direction of the connector;
FIG. 18D is a side view showing the connecting/disconnecting structure of the wire of the treatment endoscope according to the third modification of the first embodiment in a state that pieces that form steps are fitted together; and
FIG. 19 is a side view showing a connecting/disconnecting mechanism configured to connect/disconnect wires of the treatment endoscope according to a fourth modification of the first embodiment partway.

### Description of Embodiments

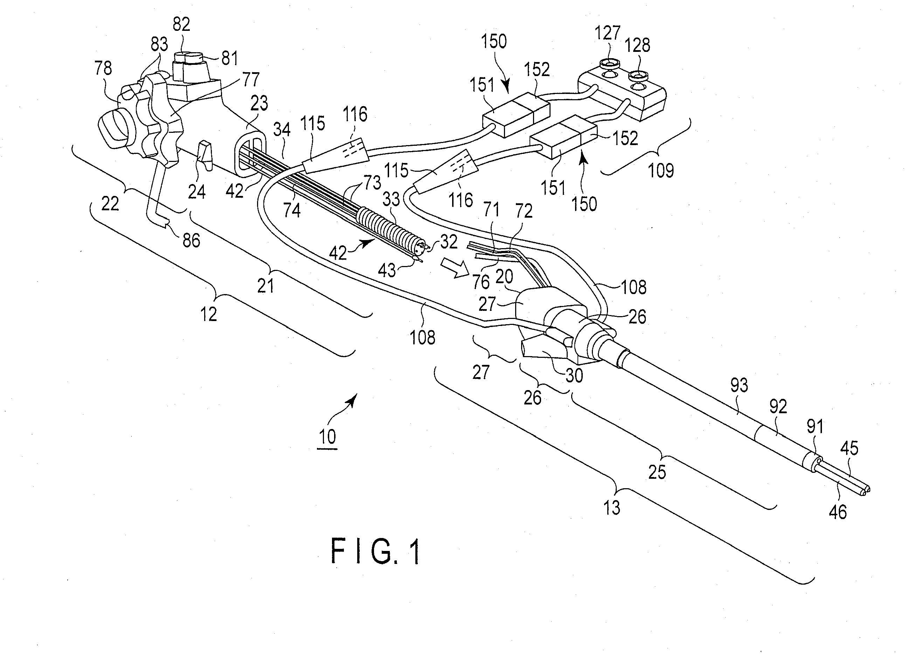

Embodiments of the present invention are described in detail below. First, a treatment endoscope according to a first embodiment is described with reference to FIG. 1 to FIG. 19.

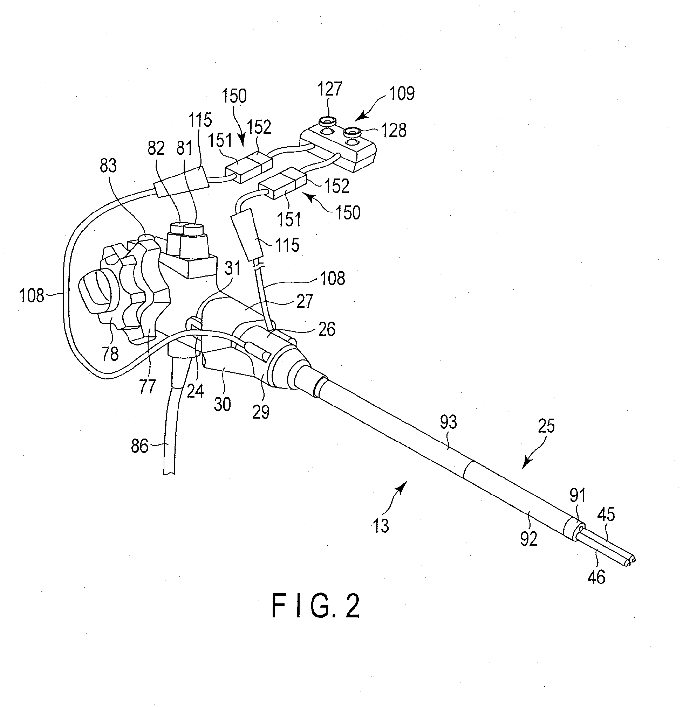

As shown in FIG. 1, a treatment endoscope 10 according to the present embodiment includes a cover-adapted endoscope 12, and an endoscope cover 13 that can be attached to the cover-adapted endoscope 12. The cover-adapted endoscope 12 includes an endoscope main body (internal main body portion) 21, and a graspable main body operation portion 22 provided to a proximal side of the endoscope main body 21. The endoscope main body 21 can be inserted into an insertion passage 20 formed in the endoscope cover 13. As shown in FIG. 2, the endoscope cover 13 is attached to the cover-adapted endoscope 12 at a predetermined position so that the endoscope main body 21 is covered with the endoscope covet 13. That is, the endoscope cover 13 is removably attached to the endoscope main body 21, and the endoscope main body 21 is covered with the attached endoscope cover 13.

As shown in FIG. 1, the endoscope cover 13 includes a cover insertion portion main body 25 which is made of a long tubular material and which is configured to be inserted into a body cavity, a distribution branch 26 disposed to the proximal side of the cover insertion portion main body 25, and a hood-shaped coupling member (coupler) 27 disposed to the proximal side of the distribution branch 26. Treatment arms 45 and 46 (described later) are provided to a distal side of the cover insertion portion main body 25.

The distribution branch 26 is fixedly attached to a proximal end of the cover insertion portion main body 25, and each internal member disposed in the endoscope cover 13 is distributed and branched to predetermined position by the distribution branch 26. The coupling member 27 includes a coupling mechanism configured to couple a side of the endoscope cover 13 to the cover-adapted endoscope 12.

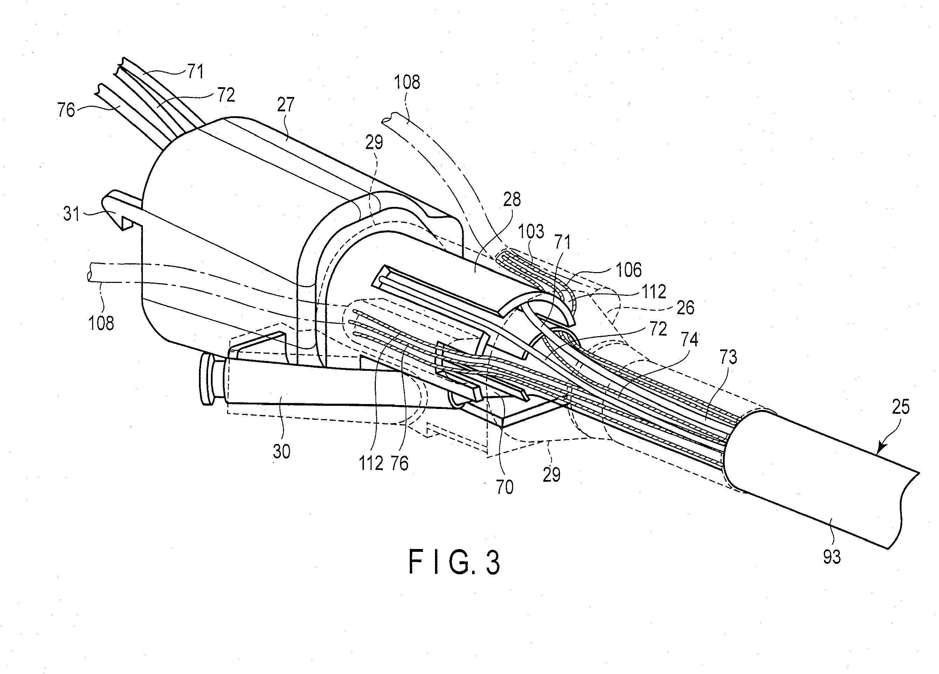

As shown in FIG. 3, the distribution branch 26 includes an exterior member 29 which is externally fitted to a cylindrical portion 28 formed at a distal end of the coupling member 27. The exterior member 29 is externally fitted to the cylindrical portion 28 so that the cylindrical portion 28 of the coupling member 27 is covered with the distribution branch 26. The exterior member 29 is provided with a treatment tool insertion hole 30 which communicates with a later-described treatment tool insertion pipe (treatment tool insertion channel) 70.

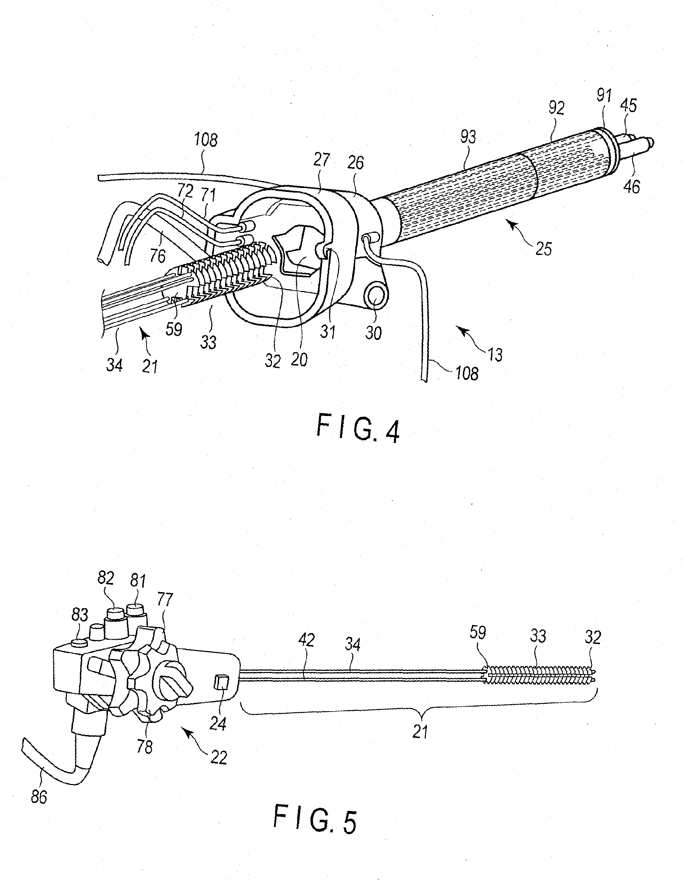

As shown in FIG. 3 and FIG. 4, a lock piece 31 is provided at a proximal edge of the coupling member 27 to project toward the proximal side (toward an endoscope hand side). The lock piece 31 detachably engages with a lock receiving portion 24 (see FIG. 1) provided on a side surface of an exterior case 23 of the main body operation portion 22, and constitutes a coupling part. The proximal end portion of the coupling member 27 is shaped to correspond to the shape of a distal end portion of the main body operation portion 22, and the coupling member 27 is fitted in close contact to the main body operation portion 22. For example, a fit portion at the proximal end portion of the coupling member 27 is formed into substantially rectangular shape in cross section, and the coupling member 27 is externally fitted to the main body operation portion 22 so that the proximal end portion of the coupling member 27 is located at a predetermined position in the axial direction and in a direction around the axis. Further, as shown in FIG. 2, the lock piece 31 and the lock receiving portion 24 engage with each other so that the coupling member 27 is externally fitted to the main body operation portion 22. As a result, the cover-adapted endoscope 12 and the endoscope cover 13 are coupled together to be held at a predetermined position. In this way, the coupling mechanism configured to removably couple the cover-adapted endoscope 12 to the endoscope cover 13 is constituted.

Now, the configuration of the cover-adapted endoscope 12 is specifically described. As described above, the cover-adapted endoscope 12 includes the endoscope main body 21 and the main body operation portion 22. As shown in FIG. 5, the endoscope main body 21 includes a main body distal portion 32 located at a most distal end side, a main body bending portion 33 located to the proximal side of the main body distal portion 32, and an internal object collection portion (flexible portion) 34 located to the proximal side of the main body bending portion 33. The main body distal portion 32, the main body bending portion 33, and the internal object collection portion 34 constitute an internal portion that can be inserted into the cover insertion portion main body 25 of the endoscope cover 13.

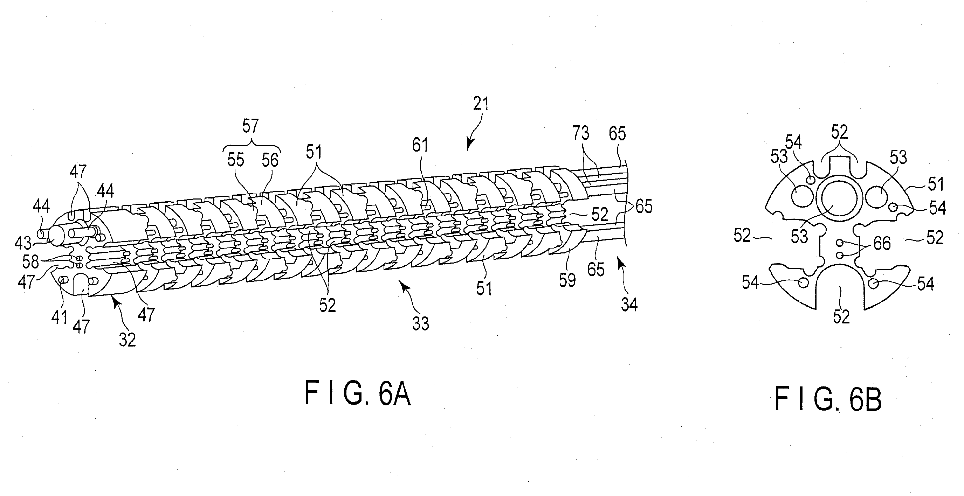

As shown in FIG. 6A, the main body distal portion 32 of the endoscope main body 21 includes a hard distal chip 41. The distal chip 41 is provided with an imaging section 43 of a later-described imaging unit (observation unit) 42, and an illumination section 44 of an illumination system. The distal chip 41 is also provided with groove portions (including bore components) 47. The groove portions 47 serve to dispose the treatment tool insertion pipe 70, a water supply tube 71, an air supply tube 72, and members that extended to the treatment arms 45 and 46, and each groove portion is located to arrange the corresponding internal object.

As shown in FIG. 6A, bending pieces 51 are arranged in the main body bending portion 33 in the direction of the longitudinal axis of the endoscope main body 21. The adjacent bending pieces 51 are coupled together rotatably relative to each other. As shown in FIG. 6B, each of the bending pieces 51 is provided with groove portions (internal object guides) 52 and bore portions (internal object guides) 53 which are configured to dispose, at predetermined positions, the internal objects that are located in the endoscope cover 13. As shown in FIG. 6A, a rear chip 59 of the main body bending portion 33 is also provided with groove portions 52 and bore portions (not shown) which are configured to dispose, at predetermined positions, the internal objects that are located in the endoscope cover 13, as in the distal chip 41.

As shown in FIG. 6A, in a coupling portion 57 of the adjacent two bending pieces 51, an engaging projection 55 is provided in one bending piece 51, and an engaging recess 56 corresponding to the engaging projection 55 is provided in the other bending piece 51. When the engaging projection 55 engages with the engaging recess 56, the adjacent two bending pieces 51 are coupled together, and the coupling portion 57 is constituted. At one end portion of each of the bending pieces 51 in the direction of the longitudinal axis of the endoscope main body 21, two engaging projections 55 are provided substantially 180° apart from each other in the circumferential direction of the endoscope main body 21. At the other end of each of the bending pieces 51 in the direction of the longitudinal axis of the endoscope main body 21, two engaging recesses 56 are provided substantially 180° apart from each other in the circumferential direction of the endoscope main body 21. In each of the bending pieces 51, the engaging projection 55 and the engaging recess 56 are located substantially 90° apart from each other in the circumferential direction of the endoscope main body 21. According to such a configuration, the coupling portions 57 each of which couples the adjacent bending pieces 51 together are formed in the main body bending portion 33 alternately at up-and-down positions and left-and-right positions of the endoscope main body 21. As a result, each of the bending pieces 51 can rotate in up-down directions and left-right directions, and the main body bending portion 33 can bend in four directions that are the up-down directions and the left-right directions.

In the main body bending portion 33, a main body bending portion coupling wire 58 configured to keep each of the bending pieces 51 in a rotatably coupled condition is extended through substantially the center of the main body bending portion 33 in the direction of the longitudinal axis. A distal end of the main body bending portion coupling wire 58 is coupled to the distal chip 41 or to a most distal side bending piece 51. A proximal end of the main body bending portion coupling wire 58 is coupled to the rear chip 59 disposed at a proximal end of the main body bending portion 33. As the bending pieces 51 are coupled together by the main body bending portion coupling wire 58 without separating in the direction of the longitudinal axis of the main body bending portion 33, the engaging projection 55 and the engaging recess 56 are firmly engaged with each other in each coupling portion 57. Moreover, bore portions 66 configured to locate the main body bending portion coupling wire 58 at a predetermined position are provided in each of the bending pieces 51.

As shown in FIG. 6B, bore portions (wire guides) 54 each of which one of four main body bending portion operating wires 61 is inserted through are provided in each of the bending pieces 51. The main body bending portion 33 is bent in the up-down directions and the left -right directions by the four main body bending portion operating wires 61. The distal ends of the main body bending portion operating wires 61 are coupled to the distal chip 41 or to the bending piece 51 fixed to the distal chip 41. Each of the main body bending portion operating wires 61 is coupled to the distal chip 41 or to the bending piece 51 fixed to the distal chip 41 at a position corresponding to the direction in which the main body bending portion 33 is bent. Each of the main body bending portion operating wires 61 is extended to the proximal side further than the rear chip 59. The distal ends of four guide sheaths 65 are coupled to the rear chip 59. Each of the main body bending portion operating wires 61 is inserted through the corresponding guide sheath 65, and guided to the main body operation portion 22 provided to the proximal side by the corresponding guide sheath 65 (see FIG. 6A). The bore portions (wire guides) 54 configured to dispose the main body bending portion operating wires 61 at predetermined positions are also provided in each of the bending pieces 51.

The internal objects that constitute the endoscope main body 21 include, for example, the treatment tool insertion pipe 70, the water supply tube 71, the air supply tube 72, a light guide 73 of the illumination system (illumination unit), an imaging cable 74 of an imaging system (an imaging unit), an arm treatment tool insertion pipe 112, and arm operation wires 103. These internal objects are disposed at the predetermined positions within the endoscope cover 13 in the main body bending portion 33 by the groove portions (internal object guides) 52 or the bore portions (internal object guides) 53. These internal objects are also distributed by the above-mentioned distribution branch 26.

As shown in FIG. 3, a proximal end of the treatment tool insertion pipe 70 is connected to the treatment tool insertion hole 30 provided in the distribution branch 26. As a result, the treatment tool insertion pipe 70 is coupled to the endoscope cover 13. The treatment tool insertion pipe 70 is provided with a connection portion (not shown) to which a suction tube 76 is connected. The treatment tool insertion pipe 70 extended from the distal side is branched, at the connection portion, into the treatment tool insertion pipe 70 extended to the treatment tool insertion hole 30 and the suction tube 76. The water supply tube 71, the air supply tube 72, and the suction tube 76 are extended to the proximal side from a proximal opening of the coupling member 27 through the distribution branch 26. The main body operation portion 22 is provided with a water supply tube connection portion, an air supply tube connection portion, and a suction tube connection portion corresponding to the water supply tube 71, the air supply tube 72, and the suction tube 76, respectively. The water supply tube 71, the air supply tube 72, and the suction tube 76 are detachably connected to the water supply tube connection portion, the air supply tube connection portion, and the suction tube connection portion by use of pipe joints, respectively.

In the cover-adapted endoscope 12, the main body bending portion operating wires 61 and each guide sheath 65 configured to guide the corresponding main body bending portion operating wire 61 are extended to the main body operation portion 22 through the internal object collection portion 34 (see FIG. 1) of the endoscope main body 21. Similarly, the imaging cable 74 of the imaging unit 42 and the light guide 73 of the illumination system (illumination unit) are extended to the main body operation portion 22 through the internal object collection portion 34. In the cover-adapted endoscope 12, the imaging unit 42 and the light guide 73 of the illumination unit are arranged side by side in the internal object collection portion 34 together with the guide sheath 65 so that one insertion unit is assembled. As a result, the imaging unit 42 and the light guide 73 can be attached to or detached from the endoscope cover 13 together with the endoscope main body 21. When the insertion unit is attached to the endoscope cover 13, the cover-adapted endoscope 12 is assembled as shown in FIG. 2. In this case, a protective member (not shown) such as a light guide protective tube that protects a distal end portion of the light guide 73 may be provided in the main body distal portion 32 of the endoscope main body 21. In this way, the distal end portion of the light guide 73 is protected by the main body distal portion 32. When the protective member is provided in the main body distal portion 32, the protective member is preferably integrally fixed to the main body distal portion 32.

In the embodiment described above, the imaging cable 74 of the imaging system and the light guide 73 of the illumination system can be attached to or detached from the endoscope cover 13. Therefore, the imaging cable 74 and the light guide 73 serve as members on the side of the endoscope main body 21 that constitute the insertion unit to be inserted into the endoscope cover 13. However, a split structure may be used to allow the imaging cable 74 of the imaging system and the light guide 73 of the illumination system to be separable partway. In this case, parts to the distal side of the separating portions of the imaging cable 74 and the light guide 73 serve as members on a side of the endoscope cover 13. Moreover, in this case, members on the side of the endoscope cover 13 in the imaging cable 74 and the light guide 73 may be configured to be integrally assembled to the endoscope cover 13. Even an internal object such as the light guide configured to guide illumination light can be provided with a separable connection portion partway. For example, the following configurations are possible: a configuration in which one light guide separation end face abuts against and is connected the other light guide separation end face, or a configuration in which the light guide separation end faces are coupled and fixed together by coupling mechanism such as rings without separating from each other via another light guide member. Alternatively, a known coupling that detachably connects light guides may be used to form a detachable connection portion. It should be understood that one separation end and the other separation end are not exclusively directly coupled together and may be indirectly connected to each other via another relay connector.

The main body operation portion 22 of the cover-adapted endoscope 12 is provided with a bending operation mechanism configured to bend the main body bending portion 33 of the endoscope main body 21. The bending operation mechanism is contained in the exterior case 23, and is driven by the operation of a up-down directions bending operation knob 77 and a left-right directions bending operation knob 78 that are provided on the side surface of the exterior case 23. When the bending operation mechanism is driven, the main body bending portion operating wires 61 are pushed or pulled in the longitudinal axis direction.

In response to the operation of the main body bending portion operating wires 61, the main body bending portion 33 is bent in the up-down directions and the left-right directions.

As shown in FIG. 1, an air supply/water supply operation button 81 and a suction operation button 82 are adjacently provided in the main body operation portion 22. The air supply/water supply operation button 81 is configured to operate a valve which controls the water supply by the water supply tube 71 and the air supply by the air supply tube 72. The suction operation button 82 is configured to operate a valve which controls the suction by the suction tube 76. An imaging system controlling switch button 83 is provided on a proximal surface of the exterior case 23 of the main body operation portion 22.

A universal cord 86 is connected to the main body operation portion 22. Members such as the water supply tube 71, the air supply tube 72, the light guide 73 made of optical fiber bundles, the imaging cable 74, and the suction tube 76 that are the above-mentioned functional members are inserted through the universal cord 86. A link connector (not shown) is provided at a proximal end portion of the universal cord 86. The link connector allows the functional members to be detachably connected to external devices such as a light source device, an air supply source, a water supply source, a suction source, and a camera control unit that are external consoles. The camera control unit converts an image signal to a video signal, and displays an observation image on a monitor (not shown).

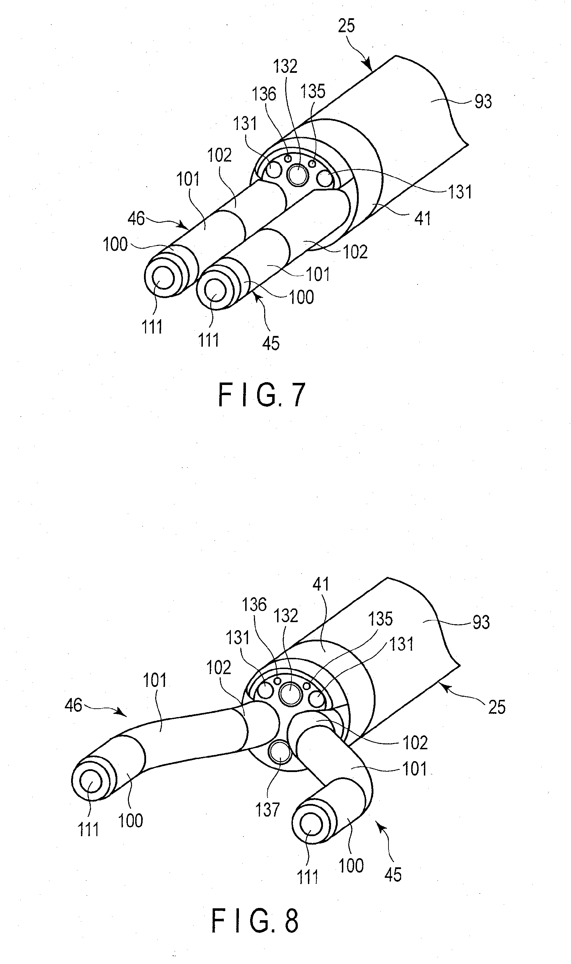

Now, the endoscope cover 13 is described in detail. As shown in FIG. 1, the cover insertion portion main body 25 of the endoscope cover 13 includes a hard portion (cover distal hard portion) 91 located at a most distal side, a flexible cover bending portion 92 which is connected to the proximal side of the hard portion 91 and which is capable of bending, and a cover flexible tube (cover flexuous tube) 93 connected to the proximal side of the cover bending portion 92. The (two in the present embodiment) treatment arms 45 and 46 project from a distal end of the cover insertion portion main body 25 toward the distal side. The proximal ends of the treatment arms 45 and 46 are coupled to the hard portion 91, and supported by the distal end of the cover insertion portion main body 25. As shown in FIG. 7, in a waiting condition, a pair of treatment arms 45 and 46 are provided side by side to linearly project from the distal end of the cover insertion portion main body 25 toward the distal side. In this case, the treatment arms 45 and 46 are located within a diametrical region of the cover insertion portion main body 25.

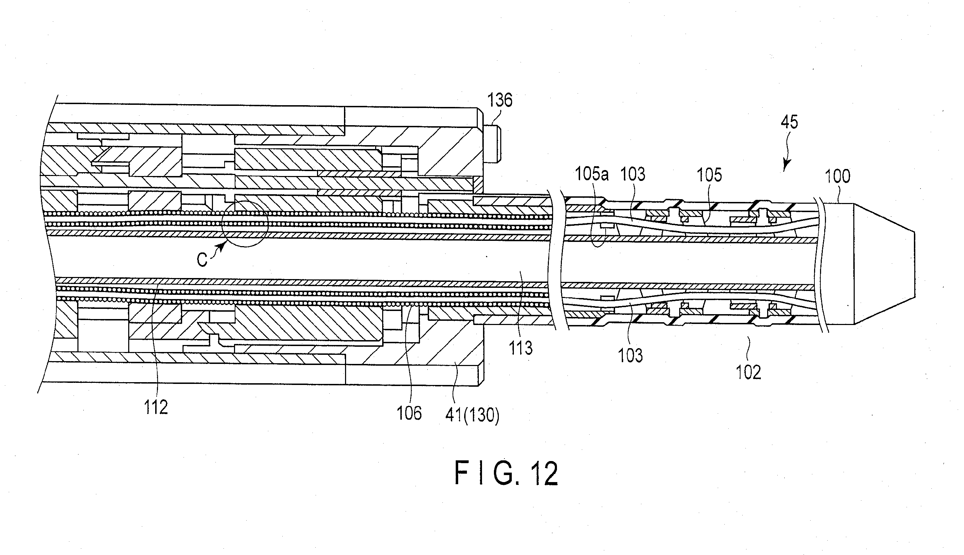

As shown in FIG. 7 and FIG. 8, each of the two treatment arms 45 and 46 includes hard portion (arm distal hard portion) 100 located at a most distal side, first bending portion (first arm bending portion) 101 connected to the proximal side of the hard portion 100, and second bending portion (second arm bending portion) 102 a distal end portion of which is connected to the proximal side of the first bending portions 101 and a proximal end portion of which is coupled to the distal end of the cover insertion portion main body 25. Each of the first bending portions 101 and the second bending portions 102 includes bending mechanism similar to a bending mechanism of a bending portion of a general endoscope. The bending mechanism of each of the of the first bending portion 101 and the second bending portion 102 includes bending pieces 105, for example, as shown in FIG. 12, and bends when the arm operation wires 103 are pulled. In the present embodiment, the first bending portion 101 located to the distal side can be bent in four directions that are up-down directions and left-right directions by the four operation wires 103 arranged to up-and-down positions and left-and-right positions. The second bending portion 102 located to the proximal side can be bent in two directions that are left-right directions by the two operation wires 103 arranged to left-and-right positions. Therefore, six operation wires 103 are required in each of the treatment arms 45 and 46, and a total of twelve operation wires 103 are used in both left-and-right treatment arms 45 and 46. The operation wires 103 are separately pushed and pulled in each of the treatment arms 45 and 46 by an operation unit 109 (described later).

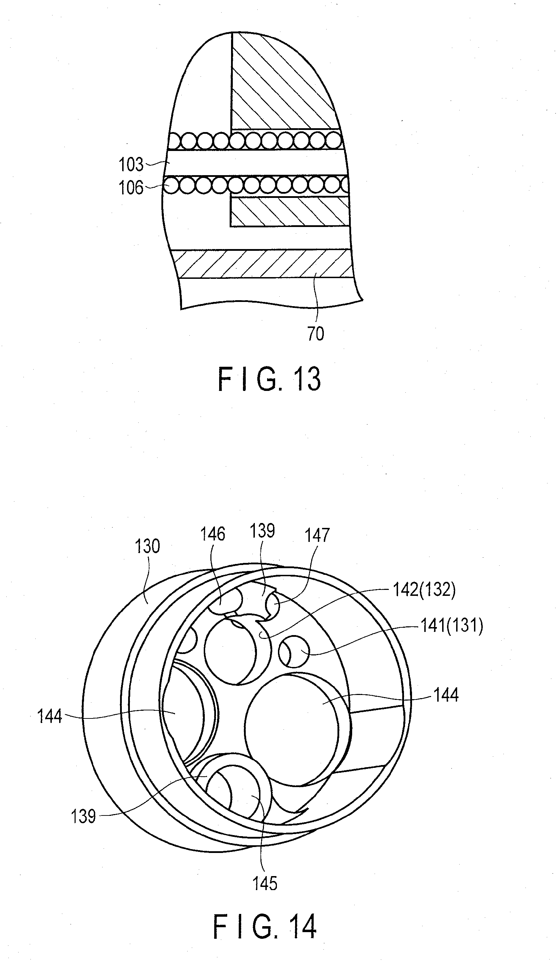

As shown in FIG. 12, in each of the first and second bending portions 101 and 102 of each treatment arm 45 and 46, the operation wires 103 are guided by guide rings 105a formed in the bending pieces 105. In the cover insertion portion main body 25, each of the operation wires 103 is guided by a corresponding guide sheath 106 made of a tightly wound coil. As shown in FIG. 12 and FIG. 13, the operation wires 103 and the guide sheaths 106 are disposed inside the endoscope cover 13. The groove portions 52 are formed in the distal chip 41, the bending pieces 51, and the rear chip 59 of the main body bending portion 33 of the endoscope main body 21. Each operation wire 103 and each guide sheath 106 are guided to the distribution branch 26 via the proximal end of the cover insertion portion main body 25 through the corresponding groove portion 52. Tubular guide cables 108 are connected to the distribution branch 26. Each of the guide sheaths 106 is guided to the arm operation unit 109 (described later) from the distribution branch 26 through the corresponding guide cable 108. In this case, the operation wires 103 are gathered into a group of operation wires 103 configures to bend the treatment arm 45 and a group of wires 103 configured to bend the treatment arm 46. The operation wires 103 of the respective groups are independently guided group by group by the two guide cables 108 corresponding to the treatment arms 45 and 46.

As shown in FIG. 7 and FIG. 8, a channel opening 111 is formed at a distal end of each of the treatment arms 45 and 46. Different channel tubes (arm treatment tool insertion tubes) 112 are connected to the respective channel openings 111 (see FIG. 12). The channel tubes 112 are longitudinally extended through the cover insertion portion main body 25. Each of the channel tubes 112 forms channel which is an arm treatment tool guide channel (a arm treatment tool insertion passage) 113 communicating with the channel opening of the corresponding treatment arm 45 and 46. Each of the channel tubes 112 passing through a region where the groove portions 52 formed in the distal chip 41, the bending pieces 51, and the rear chip 59 of the main body bending portion 33 of the endoscope main body 21 disposed in the endoscope cover 13 is extended from the corresponding treatment arm 45 and 46. Thus, each of the channel tubes 112 is guided to the distribution branch 26 via the proximal end of the cover insertion portion main body 25. Further, each of the channel tubes 112 is guided to an arm branch 115 (described later) through the guide cable 108 connected to the distribution branch 26. Each of the channel tubes 112 is guided by the guide cable 108 together with the guide sheaths 106 configured to guide the operation wires 103 of the corresponding treatment arm 45 and 46.

As shown in FIG. 1 and FIG. 2, the arm branch 115 is provided partway in each of the guide cables 108. The arm branch 115 is provided with an outwardly open arm treatment tool insertion hole 116 which communicates with the channel tube 112 guided by the guide cable 108. When an arm treatment tool is used, an arm treatment tool insertion hole 116 from which the arm treatment tool to be inserted is selected from arm treatment tool insertion holes 116. This arm treatment tool is then inserted from the selected arm treatment tool insertion hole 116. The inserted arm treatment tool is guided to the channel opening 111 of the corresponding treatment arm 45 and 46 through the channel tube 112 which communicates with the treatment tool insertion hole 116. As a result, the arm treatment tool can be used so that the arm treatment tool projects into the body cavity from the channel opening 111 of the corresponding treatment arm 45 and 46. It should be understood that the treatment tool guide channel is not only used in inserting or removing the arm treatment tool but can also be used in other purposes, for example, in supplying water into the body cavity from the distal opening of the corresponding treatment arm 45 and 46 or in injecting or sucking a fluid such as a chemical. Moreover, in the present embodiment, each of the treatment arms 45 and 46 is treatment arm in which opening that communicates with the treatment tool guide channel is formed. However, for example, as shown in a first modification in

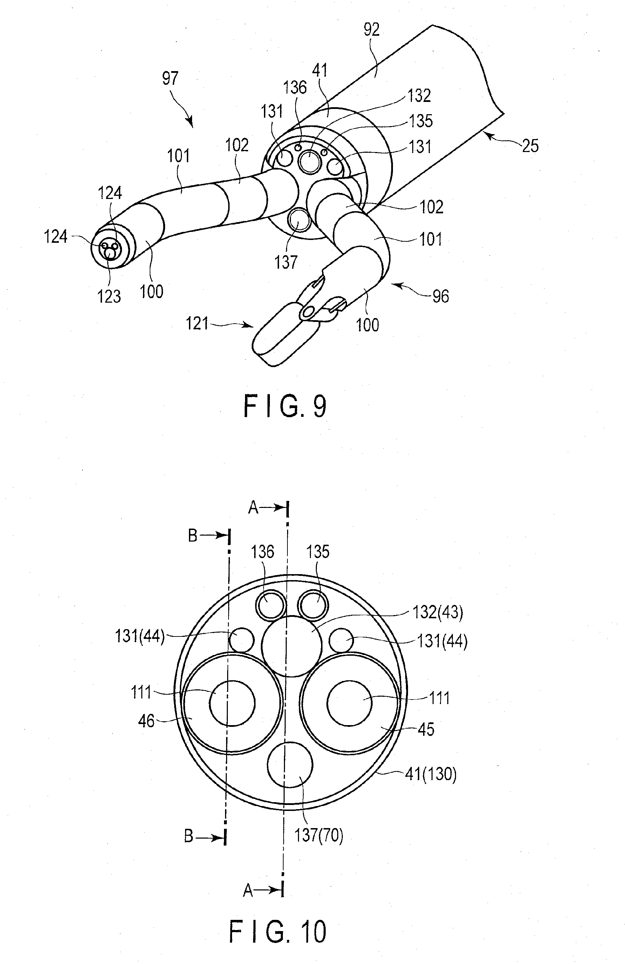

FIG. 9, a treatment portion 121 in the form of, for example, a forceps may be provided at a distal end portion of one treatment arm 96, and an observation function may be configured by providing an observation section 123 and an illumination window 124 at a distal end portion of the other treatment arm 97.

As shown in FIG. 1, a proximal end of each of the guide cables 108 is connected to the operation unit 109 through the branch 115. Each operation wire 103 configured to operate the corresponding treatment arm 45 and 46 is guided to the operation unit 109 through the corresponding guide cable 108. The operation unit 109 is provided with two operation driving mechanisms configured to separately operate the operation wires 103 corresponding to the treatment arm 45 and the operation wires 103 corresponding to the treatment arm 46. The operation unit 109 is provided with two operation handles 127 and 128 configured to separately operate the two operation driving mechanisms. Each operation handle 127 and 128 is operated to push or pull the operation wires 103 and independently bend the first arm bending portion 101 and the second arm bending portion 102 of the corresponding treatment arm 45 and 46. That is, Each operation handle 127 and 128 is operated to independently operate the first arm bending portion 101 and the second arm bending portion 102 of the treatment arm 45 and 46 corresponding to the operated operation handle 127 and 128. FIG. 7 shows a waiting condition in which both the first arm bending portions 101 and the second arm bending portions 102 of the left-and-right treatment arms 45 and 46 are not bent. FIG. 8 shows one condition during treatment operation in which the first arm bending portions 101 and the second arm bending portions 102 of the left-and-right treatment arms 45 and 46 are bent and the treatment arms 45 and 46 are outstretched in left-right directions.

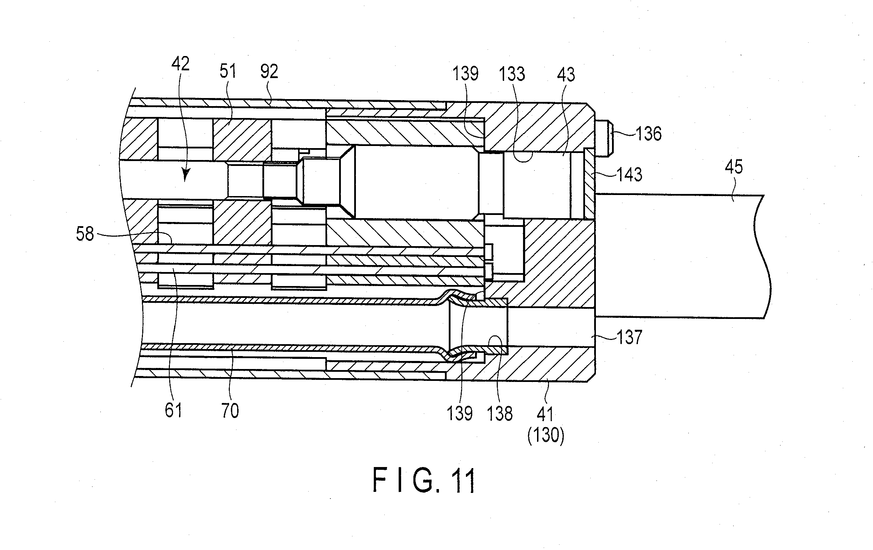

As shown in FIG. 10 to FIG. 14, a distal cover main body 130 is provided at a distal part of the cover insertion portion main body 25 of the endoscope cover 13. The first arm 45 and the second arm 46 project from a distal end face of the distal cover main body 130 toward the distal side. As shown in FIG. 10, a pair of illumination windows 131 are symmetrically arranged in the left-right directions and provided in a distal wall of the distal cover main body 130 at positions upwardly off the first arm 45 and the second arm 46. An observation window 132 is provided in a region between the pair of illumination windows 131. As shown in FIG. 11, to the proximal side of the observation window 132, an imaging section storing portion 133 is formed to fixedly dispose the imaging section 43 of the imaging unit 42. In a region upper to the observation window 132, a water supply hole 135 connected to the water supply tube 71 and an air supply hole 136 connected to the air supply tube 72 are provided. A cover main body channel hole 137 is provided in a region downwardly off the first treatment arm 45 and the second treatment arm 46. As shown in FIG. 11, a distal end of the treatment tool insertion pipe 70 is connected to the cover main body channel hole 137 via a connection cap 138.

As shown in FIG. 11, FIG. 12, and FIG. 14, an abutment portion 139 is provided on the inner wall of the distal cover main body 130. When the endoscope main body 21 is inserted in the endoscope cover 13, the distal end of the endoscope main body 21, for example, the distal chip 41 abuts against the abutment portion 139. Thus, when the endoscope main body 21 is inserted into the endoscope cover 13, the end position of the endoscope main body 21 in the axial direction is determined (see FIG. 11 and FIG. 12). That is, the abutment portion 139 constitutes a positioning mechanism configured to determine the end position in the insertion of the endoscope main body 21 into the endoscope cover 13. The positioning mechanism determines the position in the insertion of the endoscope main body 21 into the endoscope cover 13 so that the distal end of the endoscope main body 21 can not unnecessarily project from the distal end of the cover insertion portion main body 25 in an attached condition where the endoscope main body 21 is inserted in the insertion passage 20 within the endoscope cover 13. The positioning mechanism may be provided in a coupling portion between the main body operation portion 22 and the coupling member 27 of the endoscope cover 13. That is, the positioning mechanism is not exclusively provided in a distal side part of the endoscope cover 13, and for example, the abutment portion which is the positioning mechanism may be provided in a proximal side part of the endoscope cover 13. The positioning mechanism may be provided in both the distal side part and the proximal side part of the endoscope cover 13.

The imaging unit 42 and the light guide 73 are integrally assembled in the endoscope main body 21, and inserted into the distal cover main body 130 together with the endoscope main body 21. Thus, it is preferable to provide positioning mechanism to determine the insertion end positions of the imaging unit 42 and the light guide 73; for example, abutment portion that determines the insertion end positions of the imaging unit 42 and the light guide 73 is provided on the inner wall of the distal cover main body 130 or in the endoscope main body 21. The positions of the internal objects disposed in the endoscope cover 13 are regulated in the endoscope cover 13 by, for example, the clearance grooves 52 as described later.
Therefore, the position of each internal object around the axis is determined when the internal object is inserted in the endoscope cover 13. That is, not only the insertion end position of the endoscope main body 21 when inserted in the endoscope cover 13 but also the posture of the endoscope main body 21 around its axis when inserted is determined. Thus, as shown in FIG. 11, the imaging section 43 of the imaging unit 42 is positioned to correspond to the observation window 132 in the direction around the axis, and the distal end of each light guide 73 is positioned to correspond to the illumination window 131 in the direction around the axis. Abutment portions against which the distal ends of the light guides 73 abut and a abutment portion against which the distal end of the imaging section 43 of the imaging unit 42 abuts may be provided in addition to the abutment portion 139.

When the endoscope main body 21 is attached to the endoscope cover 13, the posture of the endoscope main body 21 around its axis is regulated so that each of the internal objects, disposed in the endoscope cover 13, is inserted into the corresponding clearance groove 52, the internal objects including, for example, the treatment tool insertion pipe 70, the water supply tube 71, and the air supply tube 72. As a result, an insertion guide (positioning mechanism) configured to guide the endoscope main body 21 in a predetermined posture around its axis is constituted. The efficiency in the insertion of the endoscope main body 21 into the endoscope cover 13 is improved by providing the insertion guide.

As shown in FIG. 14, the illumination window 131 and the observation window 132 are provided in through-bores 141 and 142 formed in the distal cover main body 130, respectively. As shown in FIG. 1, the distal openings of the through-bores 141 and 142 are blocked with a transparent cover member 143. The distal cover main body 130 is provided with bores 144 each of which is configured to attach a member that continue to the corresponding treatment arm 45 and 46, a bore 145 configured to attach a distal end of the treatment tool insertion pipe 70, a bore 146 configured to attach a distal end of the water supply tube 71, and a bore 147 configured to attach a distal end of the air supply tube 72.

Each of bending operation unit separating portions (arm operation portion separating portions) 150 configured to separate the corresponding guide cable 108 is provided partway in the guide cable (guide tube) 108 branched from the above-mentioned arm branch 115 and extended to the operation unit 109 (see FIG. 1 and FIG. 2). Both the bending operation unit separating portions 150 are similar in configuration. A first connector 151 is provided at one end of each of the disconnected guide cables 108, and a second connector 152 is provided at the other end. The first connector 151 and the second connector 152 can be attached to or detached from each other. The operation wires 103 included in each of the guide cables 108 can also be disconnected partway together with the connectors 151 and 152.

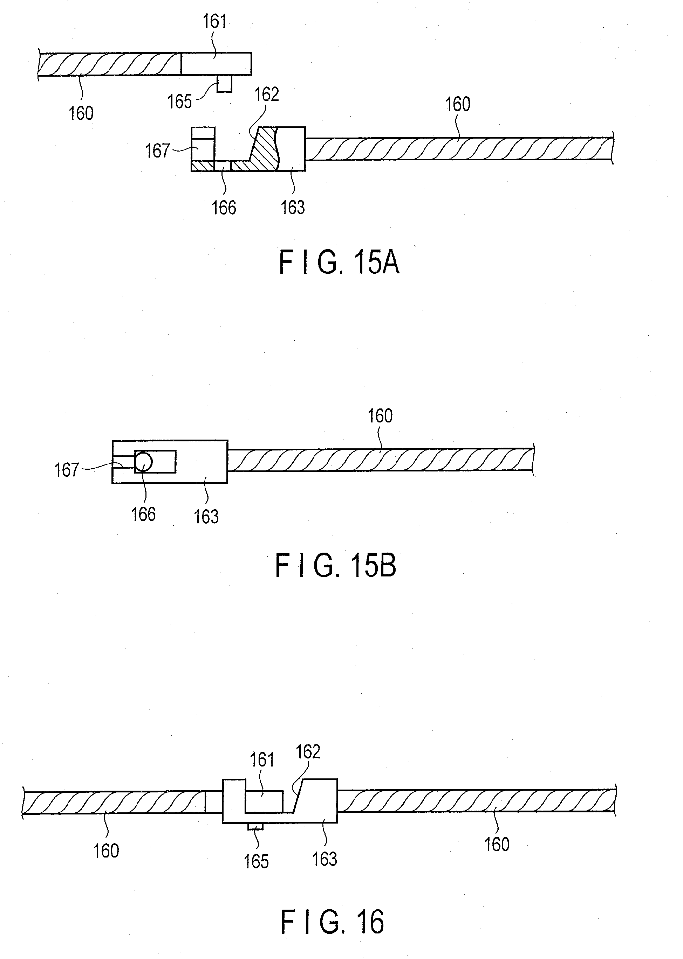

FIG. 15A to FIG. 16 are diagrams showing forms of connecting a wire 160 such as the operation wire according to the present embodiment at a separation portion provided partway in the wire. In the connection form according to the present embodiment, a columnar connection chip 161 is provided at one separation end of the wire 160 that can be disconnected partway. A connector 163 is provided at the other separation end of the wire 160. In a side surface of the connector 163, a recess 162 into which the connection chip 161 is inserted is formed. A lock pin 165 is provided on a side surface of the connection chip 161. The lock pin 165 can engage with a lock hole 166 formed in a bottom surface of the recess 162. A holding portion 167 is formed at a distal end part of the connector 163. The holding portion 167 holds the connection chip 161 when the connection chip 161 is inserted into the recess 162.

In order to connect the connection chip 161 to the connector 163, the connection chip 161 is inserted into the recess 162 from the side surface of the connector 163 where the recess 162 is formed, as shown in FIG. 15A. Further, the lock pin 165 engages with the lock hole 166, and the connection chip 161 is held by the holding portion 167, thereby coupling the connection chip 161 to the connector 163, as shown in FIG. 16.

The separated parts of the wire 160 may be connected in another form. For example, in a connection form according to an example, the connection chip 161 is inserted into the recess 162 of the connector 163 so that a distal end of the connection chip 161 is at right angles to the recess 162 of the connector 163. Further, the connection chip 161 is inserted into a predetermined position of the recess 162 by tilting the connection chip 161 so that the axial center of the connection chip 161 is aligned with the axial center direction of the connector 163. According to this connection form, the connection chip 161 can be easily coupled to the connector 163. The connection chip 161 is separated from the connector 163 by performing the coupling procedure in reverse order.

While the separated parts of one wire are connected in the connection form described above, a large number of wires can be connected or disconnected all together in a connection form described below as a second modification of the present embodiment.

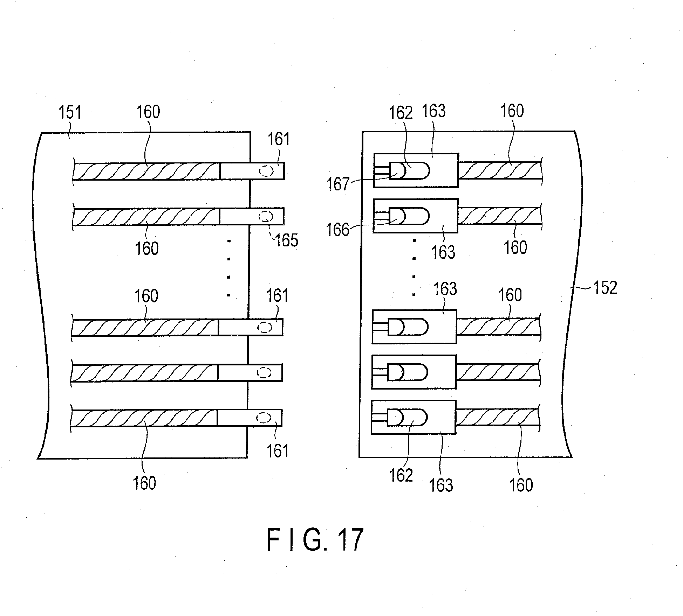

As shown in FIG. 17, according to this modification, the connection chips 161 each of which is provided in the corresponding wire 160 at one end are arranged side by side in the main body member of the first connector 151. The connectors 163, each of which is provided in the corresponding wire 160 at the other end and which are provided to correspond to the connectors 163, are arranged side by side in the main body member of the second connector 152. According to such a configuration, when the first connector 151 and the second connector 152 are connected to each other, each connection chip 161 can be attached to or detached from the corresponding connector 163 all together in the wires 160.

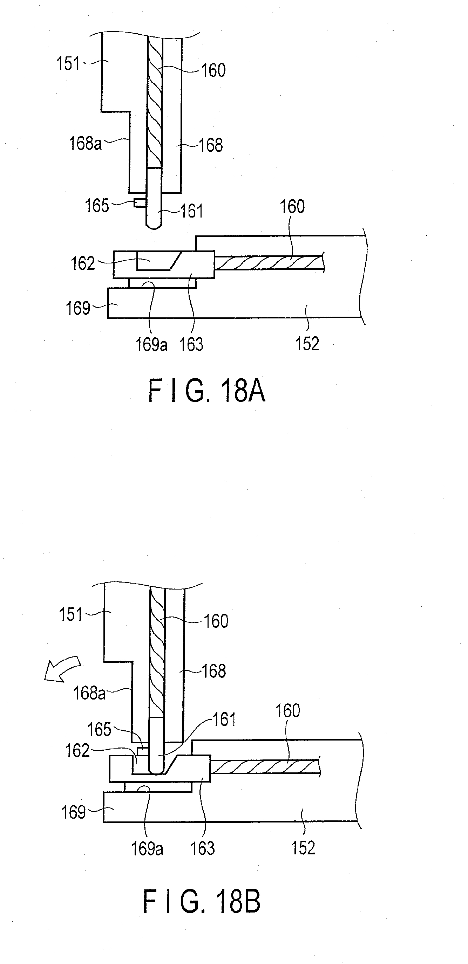

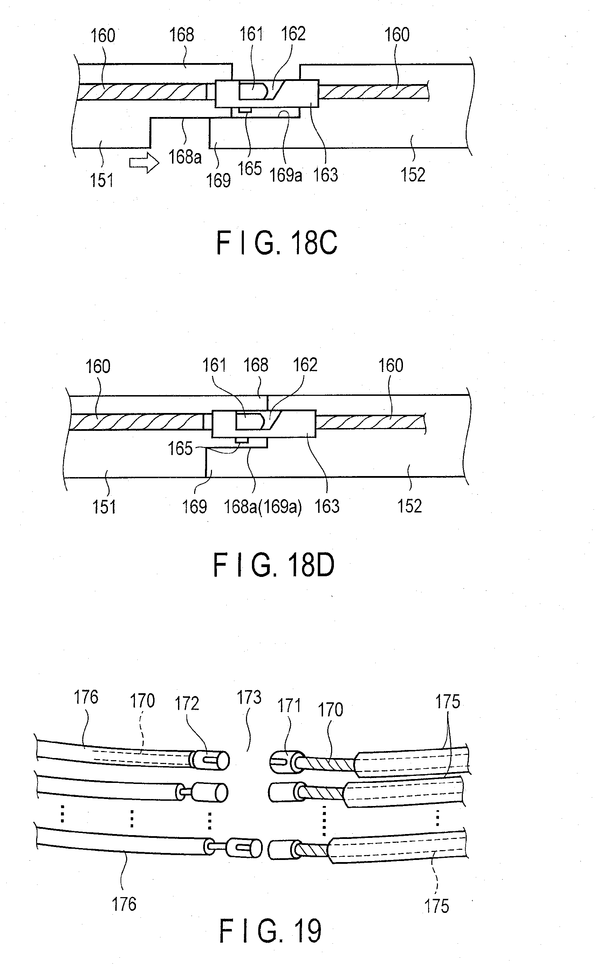

According to a third modification of the present embodiment, as shown in FIG. 18A to FIG. 18D, pieces 168 and 169 to be engaged with each other are formed in the connection portions of the first connector 151 and the second connector 152. The first connector 151 and the second connector 152 are coupled together by fitting together steps 168a and 169b that are formed in the pieces 168 and 169, as shown in FIG. 18D. A procedure of coupling the pieces 168 and 169 is shown in FIG. 18A to FIG. 18D. The connection form according to this modification is preferably applied to the connection of the wires of the above-described bending operation unit separating portions 150 at the separated part.

Various forms may be used instead of the above-described forms as the forms of connecting the wires at the separated part. For example, a first connection fitting in the form of a nut may be used in one connection fitting, and a second connection fitting in the form of an external screw may be used in the other connection fitting. In this connection form, the first connection fitting and the second connection fitting are then removably screwed together, so that a separable connection portion is formed.

FIG. 19 is a diagram showing a connecting mechanism configured to connect each wire at a separated part according to a fourth modification of the first embodiment. In this modification, a push-on turn-off joint structure is provided as a mechanism configured to connect separated part of each wire 170. In this connecting mechanism, a first connection fitting 171 is provided in a wire portion of the separated part of the wire 170 located to a insertion portion main body side. A second connection fitting 172 is provided in a wire portion of the separated part of the wire 170 located to an operation portion side. As a result, a connection portion 173 which detachably connects the first connection fitting 171 and the second connection fitting 172 is configured. In the joint structure according to this modification, one of the first connection fitting 171 and the second connection fitting 172 is inserted into the other so that both the connection fittings are automatically connected in a locked state. The locked state can be released by rotating, for example, a release ring to separate the first connection fitting 171 and the second connection fitting 172 from each other. Each wire 170 is inserted through corresponding wire sheaths (guide members) 175 and 176 except for its separated part (joint portion), and guided by the corresponding wire sheaths 175 and 176. As in the second modification shown in FIG. 17, the first connection fittings 171 may be arranged side by side in the main body member of the first connector 151 of the bending operation unit separating portion 150, and the second connection fittings 172 may be arranged side by side in the main body member of the second connector 152. As a result, a large number of wires can be connected or disconnected all together. The connection form in which the first and second connection fittings 171 and 172 are arranged side by side is preferably applied to the form of connecting the wires of the above-described bending operation unit separating portions 150 at the separated part.

A detachable connection portion may be provided to a distal side part of the arm branch 115 partway in the guide cable 108. In this way, parts to the side of the operation unit 109 including the arm branch 115 can be separated from the side of the endoscope cover 13 (the cover insertion portion main body 25).

Detachable connection portions may be provided in the extensions of the water supply tube 71 and the air supply tube 72 extended toward the proximal side from the endoscope cover 13. In this way, parts of the water supply tube 71 and the air supply tube 72 to the proximal side of the connection portions are separated from parts of the water supply tube 71 and the air supply tube 72 provided to the side of the main body operation portion 22 of the cover-adapted endoscope 12.

Separable connection portions may be provided for members such as the other internal objects depending on the kinds of members. For example, a detachable connection portion that uses a known fluid coupling (pipe joint) may be provided as a connection portion of the tube. For example, in the detachable tube connection portion, a plug is provided in one tube separation end. A socket is provided in the other tube separation end. The plug and the socket are connected and coupled together without separating from each other by putting the plug into the socket (plug-in form). Alternatively, the separation ends of two tubes may be coupled together in a screw-in form. The tube separation ends to be connected are kept firmly connected by a fastening mechanism such as a fastening ring. The connection form is not limited to directly coupling one tube separation end to the other tube separation end. The separation ends may be indirectly connected to each other via another relay connector. For an electric wire such as a signal line, a coupling mechanism may be configured so that separation ends are conductively connected to each other by a known plug and socket. In this case, a connecting mechanism configured to put the plug into the socket is constituted. The above-mentioned separable connection form of the wires may be applied to the electric wire. In this case as well, one separation end and the other separation end are not exclusively directly coupled together and may be indirectly connected to each other via another relay connector.

In the embodiment and its modifications described above, at the place to separate the side of the operation portions 22 and 109 from the side of the cover insertion portion main body 25 of the treatment endoscope 10, the connection portion is provided to detachably connect the internal object partway. However, it is not necessary for a connection portion that detachably connects the internal object partway to be provided at the place to separate the side of the operation portions 22 and 109 from the side of the cover insertion portion main body 25. For example, at the place to separate the side of the operation portions 22 and 109 from the side of the cover insertion portion main body 25, the internal object may be extended to the proximal side from the side of the cover insertion portion main body 25, and the above-mentioned separable connection portion (connecting/disconnecting portion) may be provided partway in the part of the internal object extended to the proximal side. Alternatively, the internal object may be extended to the proximal side from the side of the cover insertion portion main body 25, and a proximal end of the internal object extended to the proximal side may be detachably connected to, for example, a member or external device to be assembled to the internal object.

As shown in FIG. 1, in the treatment endoscope 10 according to the present embodiment, the cover-adapted endoscope 12 and the endoscope cover 13 are separable from each other. When the treatment endoscope 10 is used, the endoscope main body 21 of the cover-adapted endoscope 12 is inserted into the endoscope cover 13 together with the imaging unit 42 and the light guides 73 that extended toward the distal side from the main body operation portion 22. In this way, as shown in FIG. 2, the endoscope main body 21 of the cover-adapted endoscope 12 can be covered with the endoscope cover 13, thereby obtaining a usable assembly. When the arm operation unit 109 is separated from the side of the endoscope cover 13 (cover insertion portion main body 25), the first connector 151 and the second connector 152 of each of the bending operation unit separating portions (arm operation portion separating portions) 150 are coupled together, thereby connecting the arm operation unit 109 to the guide cables 108.

After the usable assembly is completed, the treatment endoscope 10 is used by inserting the cover insertion portion main body 25 into the body cavity. The main body bending portion 33 of the endoscope main body 21 is bent by the bending operation knobs 77 and 78 of the main body operation portion 22. When the main body bending portion 33 is bent, the cover bending portion 92 of the cover insertion portion main body 25 is bent. The arms 45 and 46 provided to the distal side of the cover insertion portion main body 25 of the treatment endoscope 10 are bent by the arm operation unit 109.

Each of the arms 45 and 46 projecting from the distal end of the cover insertion portion main body 25 of the endoscope cover 13 includes the arm bending portions 101 and 102 each of which has a bending mechanism separate from that of the main body bending portion 33 of the endoscope main body 21. The arm bending portions 101 and 102 can be bent independently from the main body bending portion 33 of the endoscope main body 21. Therefore, even an affected part within a complex body cavity can be easily accessed, and a complex treatment, for example, pinching and cutting off an affected part is facilitated.

Furthermore, in the treatment endoscope 10, both the arms 45 and 46 are supported on the distal end of the cover insertion portion main body 25. Thus, the arms 45 and 46 follow the motion of the distal end of the cover insertion portion main body 25. Therefore, when the cover bending portion 92 of the cover insertion portion main body 25 is bent, the arms 45 and 46 move in accordance with the motion of the distal end of the cover insertion portion main body 25. That is, the arms 45 and 46 move with reference to the distal end of the cover insertion portion main body 25. In this way, the arms 45 and 46 which are treatment operation arms are supported by the distal end of the cover insertion portion main body 25 to move in accordance with the motion of the distal end of the cover insertion portion main body 25. Therefore, the motions of the arms 45 and 46 can be recognized in the view field of the observation unit provided in the cover insertion portion main body 25. Consequently, it is possible to keep track of the arms 45 and 46 while the first arm 45 and the second arm 46 are recognized in the view field of the imaging unit by an observation mechanism. Even if the first arm 45 and/or the second arm 46 is out of the observation view field, the first arm 45 and the second arm 46 are supported on the distal end of the cover insertion portion main body 25 and can therefore be easily brought back into the observation view field. The observation of the treatment conducted by the first arm 45 and the second arm 46 can be basically continued. This facilitates and ensures the treatment in the body cavity, and allows a treatment to be rapidly and accurately conducted by the arms 45 and 46.

In the treatment endoscope 10 according to the present embodiment, the treatment arms 45 and 46 are provided on the side of the endoscope cover 13. Therefore, the efficiency in the insertion of the endoscope main body 21 of the cover-adapted endoscope 12 into the endoscope cover 13 can be easily ensured. Moreover, the endoscope cover 13 that includes the treatment arms 45 and 46 having complex configurations can be removed from the endoscope main body 21, so that the side of the endoscope cover 13 can be replaced in every treatment. Thus, cleaning in every use can be omitted or simplified. Therefore, even when treatments are conducted one after another, the time of each treatment can be reduced by replacing the side of the endoscope cover 13. Moreover, various forms of endoscope covers 13 each of which includes a cover bending portion 92 and arms 45 and 46 can be used in a common endoscope main body 21. Therefore, various forms of cover-adapted treatment endoscopes 10 can be easily obtained. That is, variations of distal end portions including the arms 45 and 46 can be selected by replacing the endoscope cover 13 in every treatment. Thus, an endoscope cover 13 is selected in accordance with the needs for a treatment, and the selected endoscope cover 13 is set to the cover-adapted endoscope 12, thereby easily constituting a desired form of a treatment endoscope 10. Consequently, a proper and effective treatment can be conducted in accordance with the needs for a treatment.

As described above, according to the treatment endoscope 10 of the present embodiment, the efficiency in the insertion of the endoscope main body 21 into the endoscope cover 13 can be ensured, and the endoscope main body 21 can be easily inserted into the endoscope cover 13 and thus used, thereby achieving the effective use of the treatment endoscope 10.

While the preferred embodiments and modifications of the present invention have been described, the present invention is not limited to the above, and these embodiments and modifications can be combined in various ways. Although a pair of treatment arms are provided in the embodiments described above, three or more treatment arms may be provided.

## Claims

1. A cover-adapted treatment endoscope (10) comprising:
an endoscope main body (21) which includes a main body distal portion (32) provided at a most distal side portion, and a main body bending portion (33) provided to a proximal side of the main body distal portion (32) and configured to bend;
a main body operation portion (22) which is provided to the proximal side of the endoscope main body (21) and which is configured to operate the main body bending portion (33); and
an endoscope cover (13) which covers the endoscope main body (21),
wherein the endoscope cover (13) includes
a cover insertion portion main body (25), the cover insertion portion main body (25) including an insertion passage (20) into which the endoscope main body (21) is inserted, a cover distal end portion (91) provided at a most distal side portion, and a cover bending portion (92) which is provided to the proximal side of the cover distal end portion (91) and which is configured to be bent in response to the bending of the main body bending portion (33) of the endoscope main body (21) in an attached condition where the endoscope main body (21) is inserted in the insertion passage (20),
a positioning mechanism (139) configured to determine an insertion position of the endoscope main body (21) where the main body distal portion (32) does not project from a distal end of the cover insertion portion main body (25) in the attached condition,
a coupling portion (27) which couples the endoscope main body (21) to the cover insertion portion main body (25) in the attached condition,
one or more treatment arm/arms (45, 46) which projects/project from the distal end of the cover insertion portion main body (25) toward a distal side and each of which includes a bending mechanism (101, 102), and
an arm operation portion (109) configured to operate the treatment arm/arms (45, 46).

2. The cover-adapted treatment endoscope (10) according to claim 1, wherein the endoscope cover (13) includes an arm operation portion separating portion (150) configured to separate the arm operation portion (109) from the cover insertion portion main body (25).

3. The cover-adapted treatment endoscope (10) according to claim 1, wherein the endoscope cover (13) includes
a treatment tool insertion hole (30, 116) which is located to the proximal side of the cover insertion portion main body (25) and into which a treatment tool is configured to be inserted,
a treatment tool projecting hole (111, 137) which is disposed in at least one of the cover distal end portion (91) of the cover insertion portion main body (25) and the treatment arm/arms (45, 46) and which is configured to project the treatment tool from the cover distal end portion (91) and/or the treatment arm/arms (45, 46) toward the distal side, and
a treatment tool insertion passage (70, 113) which allows the treatment tool insertion hole (30, 116) to communicate with the treatment tool projecting hole (111, 137).

4. The cover-adapted treatment endoscope (10) according to claim 1, wherein the endoscope cover (13) includes
a fluid outlet (135, 136) which is disposed in the cover distal end portion (91) of the cover insertion portion main body (25) and which is configured to supply air or water into a body cavity, and
a fluid supply duct (71, 72) which is configured to supply a fluid to the fluid outlet (135, 136).

5. The cover-adapted treatment endoscope (10) according to claim 1, wherein the endoscope main body (21) includes
a bending operation wire (61) which is configured to be operated by the main body operation portion (22) and which is configured to bend the main body bending portion (33), and
an internal object (70, 71, 72, 73, 74, 103, 112) inserted into the insertion passage (20) of the endoscope cover (13), and
the main body bending portion (33) includes bending pieces (51) coupled movably relative to each other,
wire guides (54) which are formed in each of the bending pieces (51) and through which the bending operation wire (61) is inserted, and
internal object guides (52, 53) which are formed in each of the bending pieces (51) and through which the internal object (70, 71, 72, 73, 74, 103, 112) is inserted.

6. An endoscope cover (13) which covers an endoscope main body (21) of a cover-adapted treatment endoscope (10), the endoscope cover (13) comprising:
a cover insertion portion main body (25), the cover insertion portion main body (25) including an insertion passage (20) into which the endoscope main body (21) is inserted, a cover distal end portion (91) provided at a most distal side portion, and a cover bending portion (92) which is provided to the proximal side of the cover distal end portion (91) and which is configured to be bent in response to the bending of a main body bending portion (33) of the endoscope main body (21) in an attached condition where the endoscope main body (21) is inserted in the insertion passage (20) ;
a positioning mechanism (139) configured to determine an insertion position of the endoscope main body (21) where the main body distal portion (32) does not project from a distal end of the cover insertion portion main body (25) in the attached condition;
a coupling portion (27) which couples the endoscope main body (21) to the cover insertion portion main body (25) in the attached condition;
one or more treatment arm/arms (45, 46) which projects/project from the distal end of the cover insertion portion main body (25) toward a distal side and each of which includes a bending mechanism (101, 102); and
an arm operation portion (109) configured to operate the treatment arm/arms (45, 46).

7. The endoscope cover (13) according to claim 6, further comprising an arm operation portion separating portion (150) configured to separate the arm operation portion (109) from the cover insertion portion main body (25).

8. The endoscope cover (13) according to claim 6, further comprising:
a treatment tool insertion hole (30, 116) which is located to the proximal side of the cover insertion portion main body (25) and into which a treatment tool is configured to be inserted;
a treatment tool projecting hole (111, 137) which is disposed in at least one of the cover distal end portion (91) of the cover insertion portion main body (25) and the treatment arm/arms (45, 46) and which is configured to project the treatment tool from the cover distal end portion (91) and/or the treatment arm/arms (45, 46) toward the distal side; and
a treatment tool insertion passage (70, 113) which allows the treatment tool insertion hole (30, 116) to communicate with the treatment tool projecting hole (111, 137).

9. The endoscope cover (13) according to claim 6, further comprising:
a fluid outlet (135, 136) which is disposed in the cover distal end portion (91) of the cover insertion portion main body (25) and which is configured to supply air or water into a body cavity, and
a fluid supply duct (71, 72) which is configured to supply a fluid to the fluid outlet (135, 136).
